# EUROPEAN PATENT SPECIFICATION

(11) **EP 2 853 194 B1**
(45) Date of publication and mention of the grant of the patent: **05.12.2018**
(21) Application number: 13187356.4
(22) Date of filing: 04.10.2013
(51) Int. Cl.: A61B 5/0408, A61B 5/00

(54) **Patches for bio-electrical signal processing**
Pflaster für bioelektrische Signalverarbeitung
Timbres pour traitement de signaux bioélectriques

(30) Priority: 30.09.2013 US 201314040744
(43) Date of publication of application: 01.04.2015
(73) Proprietor: MediaTek Inc., Hsin-Chu (TW)
(72) Inventor: Hsu, Chien-Hua, Hsinchu County (TW); Kuo, Jing-Lin, Taoyuan County (TW)
(74) Representative: Hoefer & Partner Patentanwälte mbB

(56) References cited:
- US-A- 5 341 806
- US-A1- 2004 236 202
- US-A1- 2009 112 105
- US-A1- 2011 077 497
- US-A1- 2011 279 963
- US-A1- 2013 116 520

## Description

### BACKGROUND

### 1. Technical Field

The invention relates generally to bio-electrical signal processing such as electrocardiography (ECG) signal processing, and more particularly, to patches for bio-electrical signal processing.

### 2. Related art

An ECG device may generate electrical signals that indicate a person's heart activities. Each of the signals may be referred to as an ECG lead signal. By examining the waveforms of the ECG lead signals with respect to time, a doctor may diagnose whether the person has a heart disease.

Sensor systems for ECG devices are known from the US2011/0077497A1, US2009/0112105A1 and US2011/0279963A1.

Nowadays, heart disease is becoming more prevalent and is causing health problems to countries around the world. One way to deal with this problem is to use the proposed patches that are not only easier to use but also more affordable.

### BRIEF SUMMARY

The present invention provides a patch for bio-electrical signal processing according to claim 1. The patch for bio-electrical signal processing includes a patch main body, a set of electrodes, and a lead signal generator. The patch main body has a folded state and an unfolded state. In each of the folded state and the unfolded state the patch main body is configured to be adhered to a living subject. The set of electrodes is disposed on the patch main body, and is configured to collect a set of skin voltages from the living subject. The lead signal generator is coupled to the set of electrodes, housed in the patch main body, and configured to receive the set of skin voltages from the set of electrodes and to generate a set of lead signals. The set of electrodes comprises a first electrode and a second electrode, when the patch main body is in the unfolded state the first and second electrodes are at a first distance apart, when the patch main body is in the folded state the first and second electrodes are at a second distance apart, and the first distance is greater than the second distance. The patch is marked by exactly two folding lines between the first and second electrodes that help a person change the patch main body between the unfolded state and the folded state.

Other features of the invention will be apparent from the accompanying drawings and from the detailed description which follows.

### BRIEF DESCRIPTION OF THE DRAWINGS

The invention is fully illustrated by the subsequent detailed description and the accompanying drawings.
FIG. 1 shows a schematic diagram depicting some exemplary positions on a human body for ECG electrodes to be adhered to.
FIG. 2 shows a simplified block diagram of a primary ECG patch according to an embodiment of the invention.
FIG. 3 shows schematic diagrams of the primary ECG patch of FIG. 2 in two different states according to an embodiment of the invention.
FIG. 4 shows a schematic diagram of the primary ECG patch of FIG. 2 not according to the invention.
FIG. 5 and FIG. 6 show schematic diagrams of some supplementary ECG patches according to some embodiments of the invention.
FIG. 7 shows schematic diagrams of two connection interfaces according to two embodiments of the invention.
FIG. 8 shows an exemplary block diagram of two ECG patches collaborating with each other according to an embodiment of the invention.

### DETAILED DESCRIPTION

Before introducing the embodiments of the invention, it has to be pointed out the patches presented are neither limited to serve as ECG patches nor intended to be used only for human body. Other types of bio-electrical signal processing applications, e.g. Electroencephalography (EEG), might utilize the patches of the invention. The disclosure below simply uses ECG on human body as an exemplary application as the embodiments of the invention.

Embodiments of the invention provide several ECG patches. Herein an ECG patch is defined as an adhesive patch that is configured to be adhered to a human body, and contains at least one electrode configured to collect at least one skin voltage
from the human body for the purpose of generating at least one ECG lead signal.

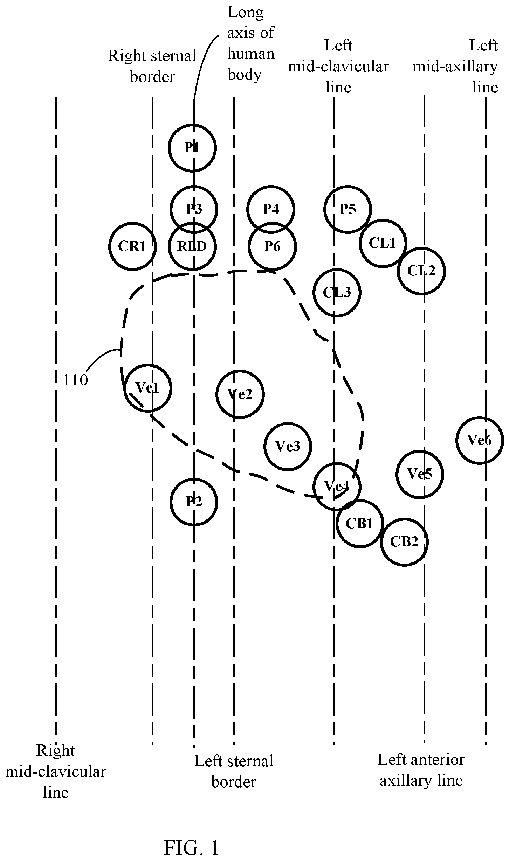

FIG. 1 shows a schematic diagram depicting some exemplary positions on a human body for ECG electrodes to be adhered to.

In FIG. 1, the enclosed broken line 110 represents the heart. Position RLD and its vicinity are exemplary positions where a right leg drive (RLD) electrode may be adhered to. This RLD electrode can decrease the common mode impedance and hence reduce the common mode noise, such like power line interference. The RLD position may be on the position of the sternal angle. When one electrode is placed thereon, other electrodes of the same ECG patch may be positioned more easily. Position CR1, Ve1, or their vicinity is where the cardio right (CR) electrode may be adhered to. This CR electrode may collect a voltage that's close to the person's right arm voltage VRA. Position CL1, CL2, CL3, or their vicinity is where a cardio left (CL) electrode may be adhered to. This CL electrode may collect a voltage that's close to the person's left arm voltage VLA. Position Ve3, Ve4, Ve5, CB1, CB2, or their vicinity is where the cardio bottom (CB) electrode may be adhered to. This CB electrode may collect a voltage that's close to the person's left leg voltage VLL. Positions Ve1, Ve2, Ve3, Ve4, Ve5, and Ve6 or their vicinity are where precordial electrodes Ve1, Ve2, Ve3, Ve4, Ve5, and Ve6 may be adhered to, respectively. The six precordial electrodes Ve1, Ve2, Ve3, Ve4, Ve5, and Ve6 are what conventionally used to generate six precordial leads V1, V2, V3, V4, V5, and V6 of a 12-lead ECG, respectively. P1, P2, P3, P4, P5, and P6 are examples of some other positions for ECG electrodes to be adhered to. Of course, an ECG electrode may be adhered to another position not specified in FIG. 1.

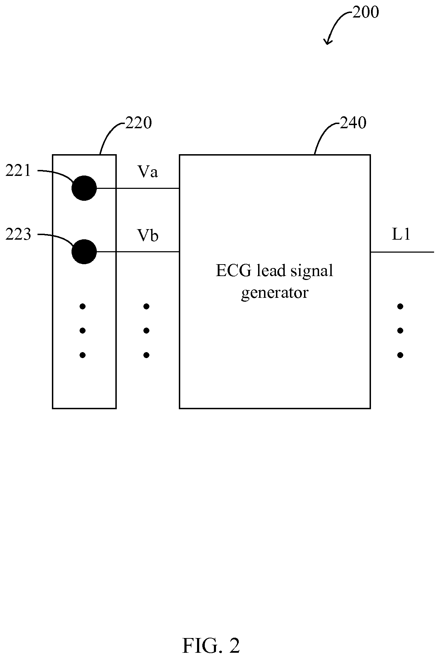

Among the ECG patches of the embodiments, an ECG patch that may function on its own without any other collaborating ECG patches may be referred to as a primary ECG patch. FIG. 2 shows a simplified block diagram of a primary ECG patch according to an embodiment of the invention. The primary ECG patch 200 has an electrode set 220 and an ECG lead signal generator 240. As used herein, a set is not an empty set, but contains at least one constituent member. One of the electrodes of the electrode set 220 may be an RLD electrode, and the other may be electrodes that are configured to acquire skin voltages.

In the example of FIG. 2, the electrode set 220 at least includes an electrode 221 and an electrode 223, configured to collect skin voltages Va and Vb from the human body, respectively. The ECG lead signal generator 240 is configured to generate at least one ECG lead signal L1 according to the skin voltages received from the electrode set 220. If the primary ECG patch 200 works on its own, the skin voltages may include only those provided by the electrode set 220. If the primary ECG patch 200 is collaborating with another ECG patch, the skin voltages may include not only those provided by the electrode set 220 but also other skin voltages provided by the collaborating ECG patch. The ECG lead signal generator 240 may include components such as amplifier and filter. For example, the ECG lead signal generator 240 may include an instrumental amplifier (IA) that generates an ECG lead signal based on the difference between the skin voltages Va and Vb.

In addition, either the ECG lead signal generator 240 or the primary ECG patch 200 may include components such as multiplexer (MUX) and analog-to-digital convertor (ADC). For example, a multiplexer may be used to selectively connect the output of one of several electrodes to an amplifier. As another example, a multiplexer may be used to selectively connect the output of one of several amplifiers to an ADC. As still another example, a multiplexer may be used to selectively connect the output of one of several ADCs to other components for further signal processing.

Moreover, the primary ECG patch 200 may further include a battery, a transceiver, and a connection interface. The battery may function as a power supply. The transceiver may transmit the ECG lead signals that the primary ECG patch 200 generates or receives to a remote device in a wireless transmission approach. For example, the remote device may be a personal computer, a laptop computer, a tablet computer, a smart phone, an access point (AP), or a telecommunication base station (BS) or Node B and the transceiver may be an RF circuit. The RF circuit and the communication network that the RF circuit collaborates with may allow the ECG lead signals to be monitored remotely. As a result, it may be determined remotely and in real time as to whether the person wearing the primary ECG patch 200 has a problem in his/her heart. As an alternative, the transmission of the ECG lead signals may be carried out through wired communication and in this case the transceiver may be a wired transceiver.

The connection interface allows another ECG patch (which may be either another primary ECG patch or a supplementary ECG patch) to be detachably connected to the primary ECG patch 200. Through the connection interface, the connected ECG patch may provide at least one ECG lead signal (if it is capable of generating it) or at least one skin voltage to the primary ECG patch 200.

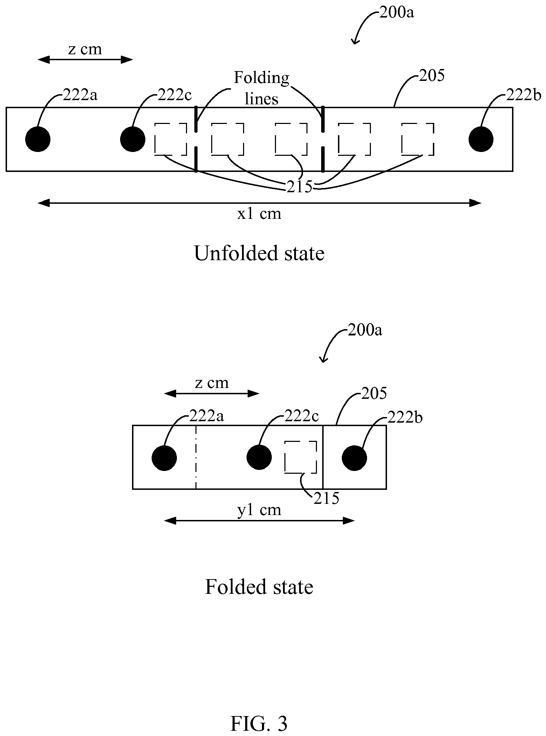

FIG. 3 shows schematic diagrams of the primary ECG patch of FIG. 2 in two different states according to an embodiment of the invention. Specifically, this primary ECG patch 200a has a foldable patch main body 205 that has an unfolded state and a folded state. Circuit components of this primary ECG patch 200a are housed in the patch main body 205. The electrode 222c may serve as either an RLD electrode or a skin voltage collection electrode, and may help positioning the patch main body 205 on the human body. For example, the electrode 222c may be adhered to the position outside the person's sternal angle. The dashed rectangles 215 depicted in FIG. 3 represent connection interfaces to be connected with other ECG patches. The patch main body 205 may have one or more of the connection interfaces. The patch main body 205 may be marked with at least one folding mark, e.g. folding line. In this example, the patch main body 205 is marked with two folding lines; they may help a person change the patch main body 205 between the unfolded state and the folded state.

When the patch main body 205 is in the unfolded state, the electrodes 222a and 222b may be placed x1 cm apart, where x1 may be greater than 12. For example, x1 may be equal to or close to 16. The electrodes 222a, 222c, and 222b may be adhered to positions CR1, RLD, and CL1 depicted in FIG. 1, respectively. As another example, electrodes 222a, 222c, and 222b may be adhered to positions Ve4, Ve3, and CR1 depicted in FIG. 1, respectively. As still another example, electrodes 222a, 222c, and 222b may be adhered to positions P1, RLD, and P2 depicted in FIG. 1, respectively.

When the patch main body 205 is in the folded state, the electrodes 222a and 222b may be placed y1 cm apart, where y1 may be less than 12. For example, y1 may be equal to or close to 8. The electrodes 222a, 222c, and 222b may be adhered to positions Ve2, Ve3, and Ve4 depicted in FIG. 1, respectively. As another example, the electrodes 222a, 222c, and 222b may be adhered to positions P3, P4, and P5 depicted in FIG. 1, respectively.

Whether the patch main body 205 is in the unfolded state or the folded state, the electrodes 222a and 222c may be placed z cm apart. For example, z may be equal to or close to 2.

Whether the patch main body 205 is in the unfolded state or the folded state, the primary ECG patch 200a may generate at least one ECG lead signal, which may at least reveal whether the person wearing the primary ECG patch 200a has arrhythmia. If more complicated diagnosis is desired, including whether the person has premature contraction, reentry, block of short path, or myocardial ischemia/infarction, the primary ECG patch 200a may collaborate with another ECG patch to provide more ECG lead signals.

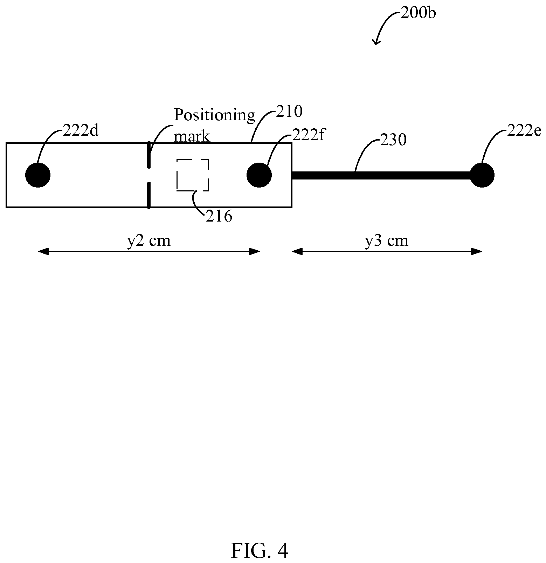

FIG. 4 shows a schematic diagram of the primary ECG patch of FIG. 2 not according to the invention. Specifically, this primary ECG patch 200b has a patch main body 210 and a movable electrode 222e connected to the patch main body 210 through a wire 230. The electrodes 222d and 222f are on the patch main body 210; circuit components of this primary ECG patch 200b are housed in the patch main body 210. The wire 230 may be adhesive or not; it allows the movable electrode 222e to be adhered several centimeters away from the patch main body 210, either on a virtual line extending through the electrodes 222d and 222f or not. As an example, the electrode 222f may serve as the RLD electrode while the electrode 222d and the movable electrode 222e may serve as skin voltage collection electrodes. Besides, the movable electrode 222e may also be adhered to the patch main body 210 instead of the human body and as an example the electrodes 222d and 222f may serve as skin voltage collection electrodes while the movable electrode 222e may not be used. The positioning mark marked on the patch main body 210 may help a person to correctly adhere the primary ECG patch 200b onto the human body. For example, the positioning mark may be aligned with the long axis of the human body, or with the upper boundary of the heart.

The electrodes 222d and 222f may be placed y2 cm apart, and the wire 230 may be y3 cm long. For example, y2 may be equal to or close to 8, and y3 may also be equal to or close to 8. The electrodes 222d, 222f, and 222e may be adhered to positions CR1, Ve2, and one of Ve3, Ve4, and Ve5 depicted in FIG. 1, respectively. As another example, electrodes 222d, 222f, and 222e may be adhered to positions CR1, P6, and one of P5, CL1, and CL2 depicted in FIG. 1, respectively.

The primary ECG patch 200b may generate at least one ECG lead signal, which may reveal at least whether the person wearing the primary ECG patch 200b has arrhythmia. If more complicated diagnosis is desired, the primary ECG patch 200b may collaborate with another ECG patch to provide more ECG lead signals through a connection interface 216.

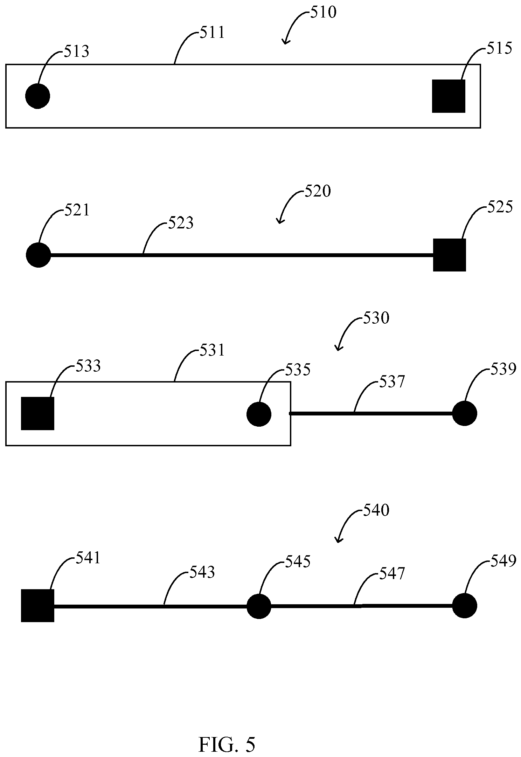

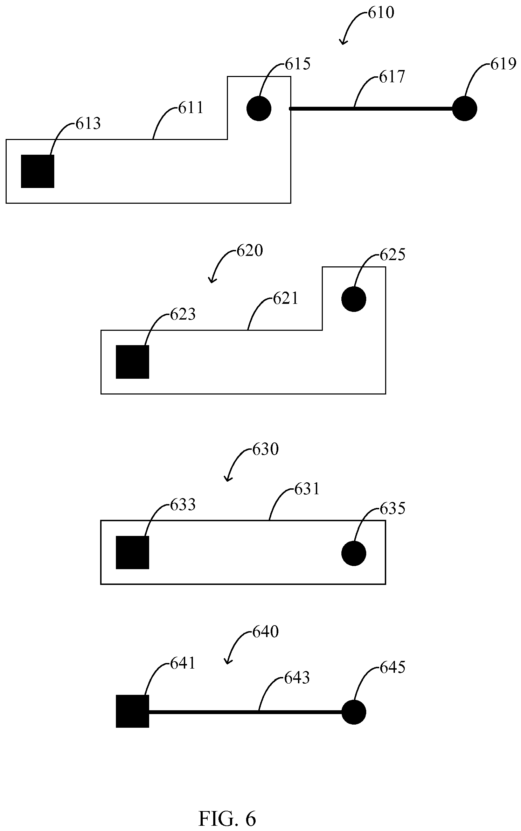

Among the ECG patches of the embodiments, an ECG patch that may not function on its own but need to collaborate with a primary ECG patch may be referred to as a supplementary ECG patch. FIG. 5 and FIG. 6 show schematic diagrams of some supplementary ECG patches according to some embodiments of the invention. Each of the supplementary ECG patches is configured to be adhered to the human body to collaborate with a primary ECG patch. Each of the supplementary ECG patches at least includes a connection interface 515, 525, 533, 541, 613, 623, 633, 641 (represented by a black rectangle) and at least an electrode 513, 521, 535, 539, 545, 549, 615, 619, 625, 635, 645 (represented by a black circle). An electrode or connection interface not located on a patch main body may be connected to another electrode/connection interface or a patch main body through a wire (represent by a thick line).

The connection interface 515, 525, 533, 541, 613, 623, 633, 641 may allow the supplementary ECG patch to be detachably connected to a primary ECG patch and transmits at least one skin voltage or at least one ECG lead signal to the primary ECG patch. To collect a skin voltage, the supplementary ECG patch needs to include an electrode. To generate an ECG lead signal, the supplementary ECG patch may need to include an amplifier.

If the supplementary ECG patch does not contain a battery, the connection interface 515, 525, 533, 541, 613, 623, 633, 641 may further allow the supplementary ECG patch to receive electricity from the primary ECG patch through the connection interface 515, 525, 533, 541, 613, 623, 633, 641. In addition, the connection interface 515, 525, 533, 541, 613, 623, 633, 641 may allow the primary ECG patch to pass a synchronization clock signal to the supplementary ECG patch if there is such a need. Depending on the amount of signals to be conveyed, the connection interface 515, 525, 533, 541, 613, 623, 633, 641 may have several conductive areas thereon. For example, the conductive areas may form concentric circles.

Please refer to FIG. 5 first. The supplementary ECG patch 510 includes a patch main body 511, an electrode 513, and a connection interface 515. The supplementary ECG patch 520 includes an electrode 521, a wire 523, and a connection interface 525. The supplementary ECG patch 530 includes a patch main body 531, a connection interface 533, an electrode 535, a wire 537, and an electrode 539. The supplementary ECG patch 540 includes a connection interface 541, a wire 543, an electrode 545, a wire 547, and an electrode 549. Please refer to FIG. 6. The supplementary ECG patch 610 includes an L-shaped patch main body 611, a connection interface 613, an electrode 615, a wire 617, and an electrode 619. The supplementary ECG patch 620 includes an L-shaped patch main body 621, a connection interface 623, and an electrode 625. The supplementary ECG patch 630 includes a patch main body 631, a connection interface 633, and an electrode 635. The supplementary ECG patch 640 includes a connection interface 641, a wire 643, and an electrode 645. Each of these supplementary ECG patch may collaborate with a primary ECG patch once the supplementary ECG patch's connection interface is connected with the primary ECG patch's connection interface.

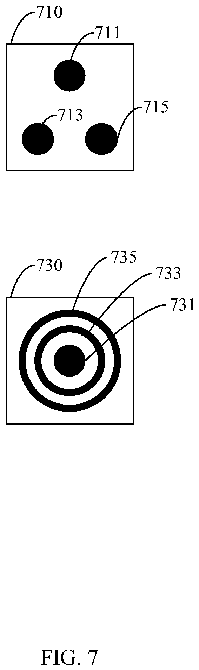

FIG. 7 shows exemplary schematic diagrams of two connection interfaces according to two embodiments of the invention. Connection interface 710 has three conductive areas 711, 713, and 715. Each of the conductive areas may convey one signal (e.g. a skin voltage, an ECG lead signal, a synchronization clock signal, or a supply voltage). With this type of connection interface, two ECG patches may be interconnected, but the angle between the two ECG patches as connected may not be changed freely. Connection interface 730 has a conductive circle 731, and two conductive rings 733 and 735; they are like concentric circles. Each of them may convey one signal. With this type of connection interface, two ECG patches may be interconnected, and the angle between the two ECG patches as connected may be changed relatively more freely. A connection interface may also be like a headphone plug in order to be detachably connected to another connection interface that's like a headphone jack.

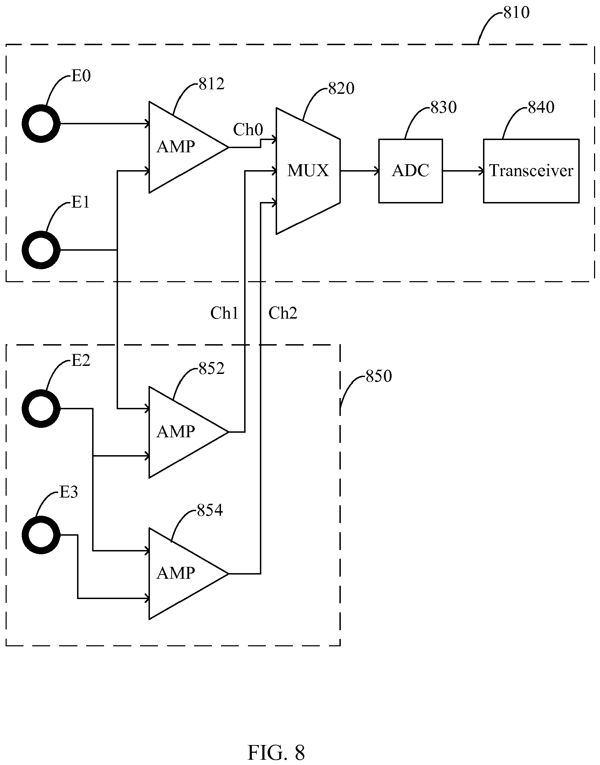

FIG. 8 shows an exemplary block diagram of two ECG patches collaborating with each other according to an embodiment of the invention. The two ECG patches are an ECG patch 810 and an ECG patch 850, the former is a primary ECG patch and the latter is either a primary ECG patch or a supplementary ECG patch. The ECG patch 810 includes electrodes E0 and E1 as a set of electrodes, and an amplifier 812 that constitutes an ECG lead signal generator. The amplifier 812 is configured to generate an ECG lead signal Ch0 based on the difference between the skin voltages E0 and E1. The ECG patch 850 includes electrodes E2 and E3 as a set of electrodes, and amplifiers 852 and 854 that constitute an ECG lead signal generator. The amplifier 852 is configured to generate an ECG lead signal Ch1 based on the difference between the skin voltages E1 and E2. The amplifier 854 is configured to generate an ECG lead signal Ch2 based on the difference between the skin voltages E2 and E3. A connection interface of the ECG patch 810 and a connection interface of the ECG patch 850, both are not depicted in FIG. 8, connect the two ECG patches 810 and 850 together.

The ECG patch 810 further includes a MUX 820, an ADC 830, and a transceiver 840. The MUX 820 selectively passes one of the ECG lead signals Ch0, Ch1, and C2 to the ADC 830. The ADC 830 converts the received ECG lead signal into digital form. The transceiver 840 processes the digitalized ECG lead signal and then transmits the processed ECG lead signal to a remote device. With this structure, the ECG patches 810 and 850 allow three ECG lead signals to be monitored remotely. Note that the processing of the digitalized ECG lead signal in the transceiver 840 may include techniques such as data compression, cryptography and digital-to-analog conversion but are not described in detail here for the sake of brevity.

The modular ECG patches of the embodiments may be combined in several different ways. Without complicated components, each of the ECG patches may be relatively more affordable. With relatively low prices, the ECG patches may even be disposable. A person may purchase only those ECG patches according to his/her actual need. As a result, the overall costs of the purchased ECG patches may be much lower than a multifunctional ECG device. Although such a multifunctional ECG device may provide more information, some of (or even most of) the information may not be needed. It may be a waste of money to purchase such an expensive multifunctional ECG device when only some of its functions are desired.

Another advantages of the ECG patches is that they are quite user friendly. A person without medical training may easily attach one or several ECG patches of the embodiments onto a human body. In addition, the person wearing the ECG patches needs not to stay in a hospital, but may be able to do most of the regular activities. Although not in a hospital, the person's heart condition may be monitored remotely, and even in real time. Because the ECG patches are very small, the person may wear the ECG patches secretly. Other people may not notice that the person is wearing the ECG patches.

In the foregoing detailed description, the invention has been described with reference to specific exemplary embodiments thereof. It will be evident that various modifications may be made thereto without departing from the scope of the invention as set forth in the following claims. The detailed description and drawings are, accordingly, to be regarded in an illustrative sense rather than a restrictive sense.

## Claims

1. A patch (200, 810) for bio-electrical signal processing, comprising:
a patch main body (205), having a folded state and an unfolded state, in each of the folded state and the unfolded state the patch main body is configured to be adhered to a living subject;
a set of electrodes (220, E0, E1), disposed on the patch main body (205), configured to detect a set of skin voltages from the living subject; and
a lead signal generator (240), coupled to the set of electrodes (220), housed in the patch main body (205), configured to receive the set of skin voltages from the set of electrodes and to generate a set of lead signals, and
wherein the set of electrodes (220) comprises a first electrode (222a) and a second electrode (222b), when the patch main body (205) is in the unfolded state the first and second electrodes (222a, 222b) are at a first distance (x1) apart, when the patch main body is in the folded state the first and second electrodes (222a, 222b) are at a second distance apart (y1), and the first distance (x1) is greater than the second distance (y1),
**characterized in that**
the patch main body (205) is marked by exactly two folding lines between the first and second electrodes (222a, 222b) that help a person change the patch main body (205) between the unfolded state and the folded state.

2. The patch for bio-electrical signal processing of claim 1, wherein the first distance (x1) is at least 2 cm greater than the second distance (y1).

3. The patch for bio-electrical signal processing of claim 1, further comprising a connection interface disposed on the patch main body (205), configured to be detachably connected to a second patch (850).

4. The patch for bio-electrical signal processing of claim 3, wherein the lead signal generator (240) is further configured to selectively receive a second set of skin voltages from the second patch through the connection interface.

5. The patch for bio-electrical signal processing of claim 1, further comprising a transmitter (840) configured to transmit the set of lead signals to a remote device.

6. The patch for bio-electrical signal processing of claim 1, further comprising a movable electrode (222e) and a wire (230), the wire (230) interconnecting the movable electrode (222e) and the patch main body (205).

## Patentansprüche

1. Pflaster (200, 810) für eine bioelektrische Signalverarbeitung, umfassend:
einen einen gefalteten Zustand und einen ungefalteten Zustand aufweisenden Pflasterhauptkörper (205), wobei der Pflasterhauptkörper in sowohl dem gefalteten Zustand als auch dem ungefalteten Zustand dafür eingerichtet ist, an ein lebendes Subjekt geklebt zu werden;
einen Satz auf dem Pflasterhauptkörper (205) angeordnete Elektroden (220, E0, E1), die dafür eingerichtet sind, einen Satz Hautspannungen von dem lebenden Subjekt zu detektieren; und
einen mit dem Satz Elektroden (220) gekoppelten, im Pflasterhauptkörper (205) untergebrachten Leitungssignalgenerator (240), der dafür eingerichtet, den Satz Hautspannungen von dem Satz Elektroden zu empfangen und einen Satz Leitungssignale zu erzeugen, und
wobei der Satz Elektroden (220) eine erste Elektrode (222a) und eine zweite Elektrode (222b) umfasst, wenn der Pflasterhauptkörper (205) im ungefalteten Zustand ist, die erste Elektrode (222a) und die zweite Elektrode (222b) in einer ersten Distanz (x1) voneinander getrennt sind, wenn der Pflasterhauptkörper im gefalteten Zustand ist, die erste Elektrode (222a) und die zweite Elektrode (222b) in einer zweiten Distanz (y1) voneinander getrennt sind und die erste Distanz (x1) größer als die zweite Distanz (y1) ist,
**dadurch gekennzeichnet, dass**
der Pflasterhauptkörper (205) durch exakt zwei Faltlinien zwischen der ersten Elektrode (222a) und der zweiten Elektrode (222b) markiert ist, die einer Person dabei helfen, den Pflasterhauptkörper (205) zwischen dem ungefalteten Zustand und dem gefalteten Zustand zu wechseln.

2. Pflaster für eine biolelektrische Signalverarbeitung nach Anspruch 1, wobei die erste Distanz (x1) mindestens 2 cm größer als die zweite Distanz (y1) ist

3. Pflaster für eine biolelektrische Signalverarbeitung nach Anspruch 1, ferner umfassend eine auf dem Pflasterhauptkörper (205) angeordnete Verbindungsschnittstelle, die dafür eingerichtet ist, mit einem zweiten Pflaster (850) lösbar verbunden zu werden.

4. Pflaster für eine biolelektrische Signalverarbeitung nach Anspruch 3, wobei der Leitungssignalgenerator (240) ferner dafür eingerichtet ist, einen zweiten Satz Hautspannungen von dem zweiten Pflaster durch die Verbindungsschnittstelle selektiv zu empfangen.

5. Pflaster für eine biolelektrische Signalverarbeitung nach Anspruch 1, ferner umfassend einen Sender (840), der dafür eingerichtet ist, den Satz Leitungssignale an eine entfernte Vorrichtung zu senden.

6. Pflaster für eine biolelektrische Signalverarbeitung nach Anspruch 1, ferner umfassend eine bewegliche Elektrode (222e) und einen Draht (230), wobei der Draht (230) die bewegliche Elektrode (222e) und den Pflasterhauptkörper (205) miteinander verbindet.

## Revendications

1. Patch (200, 810) de traitement de signaux bioélectriques, comprenant:
un corps principal de patch (205), ayant un état plié et un état déplié, dans chacun des états plié et déplié, le corps principal de patch est configuré pour être collé à un sujet vivant ;
un ensemble d'électrodes (220, E0, E1), disposé sur le corps principal de patch (205), configuré pour détecter un ensemble de tensions de la peau provenant du sujet vivant ; et
un générateur de signaux de dérivation (240), couplé à l'ensemble d'électrodes (220), logé dans le corps principal de patch (205), configuré pour recevoir l'ensemble des tensions de la peau à partir de l'ensemble d'électrodes et pour générer un ensemble de signaux de dérivation, et
dans lequel l'ensemble d'électrodes (220) comprend une première électrode (222a) et une deuxième électrode (222b), lorsque le corps principal de patch (205) est dans l'état déplié, les première et deuxième électrodes (222a, 222b) sont espacées d'une première distance (x1), lorsque le corps principal de patch est dans l'état plié, les première et deuxième électrodes (222a, 222b) sont espacées d'une deuxième distance (y1), et la première distance (x1) est supérieure à la deuxième distance (y1),
**caractérisé en ce que**
le corps principal de patch (205) est marqué par exactement deux lignes de pliage entre les première et deuxième électrodes (222a, 222b) qui aident une personne à faire passer le corps principal de patch (205) de l'état déplié vers l'état plié.

2. Patch de traitement de signaux bioélectriques de la revendication 1, dans lequel la première distance (x1) est supérieure d'au moins 2 cm à la deuxième distance (y1).

3. Patch de traitement de signaux bioélectriques de la revendication 1, comprenant en outre une interface de liaison disposée sur le corps principal de patch (205), configurée pour être reliée de manière détachable à un deuxième patch (850).

4. Patch de traitement de signaux bioélectriques de la revendication 3, dans lequel le générateur de signaux de dérivation (240) est en outre configuré pour recevoir sélectivement un deuxième ensemble de tensions de la peau provenant du deuxième patch à travers l'interface de liaison.

5. Patch de traitement de signaux bioélectriques de la revendication 1, comprenant en outre un émetteur (840) configuré pour transmettre l'ensemble de signaux de dérivation à un dispositif distant.

6. Patch de traitement de signaux bioélectriques de la revendication 1, comprenant en outre une électrode mobile (222e) et un fil (230), le fil (230) reliant l'électrode mobile (222e) et le corps principal de patch (205).
